# EUROPÄISCHE PATENTSCHRIFT

(11) **EP 2 542 873 B1**
(45) Veröffentlichungstag und Bekanntmachung des Hinweises auf die Patenterteilung: **15.07.2015**
(21) Anmeldenummer: 11704787.8
(22) Anmeldetag: 23.02.2011
(51) Int. Cl.: G01N 1/20

(54) **EINRICHTUNG ZUR ENTNAHME VON PROBEN AUS EINEM PULVERSTROM**
DEVICE FOR TAKING SAMPLES FROM A POWDER STREAM
DISPOSITIF POUR PRÉLEVER DES ÉCHANTILLONS DANS UN FLUX DE POUDRE

(30) Priorität: 17.03.2010 DE 102010011724
(43) Veröffentlichungstag der Anmeldung: 09.01.2013
(73) Patentinhaber: Glatt Systemtechnik GmbH, 01277 Dresden (DE)
(72) Erfinder: PRITZKE, Heinz, 01737 Braunsdorf (DE)
(74) Vertreter: Pätzelt, Peter
(86) Internationale Anmeldenummer: PCT/EP2011/052643
(87) Internationale Veröffentlichungsnummer: WO 2011/113670

(56) Entgegenhaltungen:
- DD-A3- 160 734
- JP-A- 7 110 291
- US-A- 3 949 614
- US-A- 4 655 371
- US-A- 4 771 642

## Beschreibung

Die Erfindung betrifft eine Einrichtung zur Entnahme von Proben aus einem insbesondere frei fallenden Pulver- oder Granulatstrom, nachfolgend kurz Pulverstrom genannt, mit den Merkmalen nach Anspruchs 1. Die Produktproben sind insbesondere zur Prüfung der aktuellen Mischungsverhältnisse sowie der Homogenität eines Mischproduktes bestehend aus mindestens zwei einzelnen pulver- oder granulatartigen, insbesondere für pharmazeutischen oder chemischen Stoffen erforderlich.

Nach dem Stand der Technik sind verschiedene Einrichtungen zur Entnahme von Produktproben aus einem frei fallenden Pulverstrom bekannt. Dabei werden in der Praxis die Produktproben meist periodisch innerhalb eines Produktionszyklus zur Prüfung vorgegebener Parameter entnommen.

Die vorliegende Erfindung betrifft dabei eine Einrichtung in der Art entsprechend des Inline-Pulverprobennehmers "SamFreeGlide" der Fa. Kerting GmbH, Brilon (DE)-(http://www.kersting-ind.de/de/produkte/probenahme/inline-probenehmer/schuettgut-probenehmer/samfreeglide.html). Der Inline-Pulverprobennehmer wird an das Schüttgutrohr angeflanscht. Eine Probentasse an einer Haltestange ist in der Ruhestellung innerhalb des Inline-Pulverprobennehmers gelagert. Am Ende der Haltestange ist eine Dichtplatte vorgesehen, die den Innenraum des Inline-Pulverprobennehmers gegenüber dem Schüttgutrohr abschließt. Zur Entnahme einer Probe wird die Haltestange mit der Probentasse und der Dichtplatte manuell oder pneumatisch in das Innere des Schüttgutrohres geschoben und, nachdem die Probentasse mit dem Schüttgut gefüllt ist, wieder zurückgezogen. Danach wird die Probentasse durch Drehung der Haltestange über einem Behältnis oder einer Rohrleitung entleert. Die Probe gelangt auf beliebigen Weg zur Analysestation. Bei einer derartigen Probenentnahme kann es dazu kommen, dass sich die entnommene Probe bei der Entleerung mindestens teilweise entmischt und es somit zu Fehlanalysen kommt.

Die DE 2456643 A1 beschreibt einen volumetrischen Probenehmer für fließbare, insbesondere körnige oder pulverförmige Stoffe in einer Prüfzone. Der Probennehmer ist radial an einem Förderrohr angeordnet und besteht aus einem Stecher mit koaxialen Futter (Verschluss), in dem eine zylindrische Sonde axial geführt ist. Die Sonde weist eine Ausnehmung auf, in der ein bestimmtes Volumen eines im Förderrohr fließenden Stoffes aufgenommen werden kann. Durch axiale Verschiebung kann die Ausnehmung sowohl in das Förderrohr als auch außerhalb desselben über einen Trichter bewegt werden. Nach der Befüllung der Sonde im Förderrohr kann die Sonde durch geeignete Mittel axial aus dem Förderrohr entfernt und über einen Trichter bewegt werden. Nach Drehung der Sonde, so dass die Ausnehmung nach unten offen ist, wird die entnommene Stoffprobe in den Trichter entleert.

Die US3949614, DD160734, JP7110291 und US4771642 offenbaren Entnahmeeinrichtungen gemäß der Präambel des Anspruchs 1.

Der Erfindung liegt damit als **Aufgabe** zugrunde, eine Einrichtung zur Entnahme von Proben aus einem Pulver- oder Granulatstrom der eingangs genannten Art anzugeben, bei der die Proben sich bis zur Analysestation nicht entmischen können. Weiterhin besteht die Aufgabe, die Menge der zu entnehmenden Probe, z.B. 1cm³, 2cm³ oder 4cm³, an den spezifischen Verfahrenszyklus anzupassen sowie den Ort der Probenentnahme innerhalb des Granulatstromes zu variieren. Weiterhin soll die Einrichtung ohne Demontage eine Reinigung des Innenraumes ermöglichen.

Die Erfindung löst die Aufgabe durch eine Einrichtung mit den im Anspruch 1 angegebenen Merkmalen. Vorteilhafte Weiterbildungen der Erfindung sind in den Unteransprüchen gekennzeichnet und werden nachstehend zusammen mit der Beschreibung der bevorzugten Ausführung der Einrichtung, einschließlich der Zeichnung, näher dargestellt.

Erfindungsgemäß ist ein Probenbehälter vorgesehen, in dem die Probe aufgenommen und ohne dass die Probe selbst bewegt oder gerüttelt wird, bis zur Analysestation gebracht werden kann.

In einer als solche bekannten Art und Weise besteht die Einrichtung aus einem Gehäuse mit einer Achse, welches radial zur Längsachse der Pulverleitung an dieser angeordnet ist. Die Stirnfläche des Gehäuses reicht bis in die Ebene der Innenwandung der Pulverleitung, damit der Pulverstrom innerhalb der Pulverleitung nicht durch Absätze behindert wird. Dabei ist es unwesentlich, ob das Gehäuse mit seiner Stirnseite unmittelbar die Wandung der Pulverleitung durchdringt oder ob ein gesondertes flanschartiges Zwischenstück in die Wandung der Pulverleitung eingebracht ist und das Gehäuse an einem äußeren Flansch des Zwischenstückes angeordnet ist.

In der Achse des Gehäuses ist verschiebbar ein Schieber gelagert. Dessen Stirnfläche liegt in der Ruhestellung ebenfalls bündig zur Innenwandung der Pulverleitung sowie der Stirnfläche des Gehäuses. Am Umfang des Schiebers, zum Pulverstrom oben, ist eine Abflachung vorgesehen, mit der eine Verdrehung gegenüber dem Gehäuse verhindert wird. Innerhalb dieser Fläche ist an der Oberseite des Schiebers eine Probenmulde vorgesehen, in die der Probenbehälter eingesetzt werden kann. Eine Schubeinrichtung ist geeignet, den Schieber in der Gehäuseachse zu verschieben, derart dass die Probenmulde mit dem Probenbehälter in den Pulverstrom bewegt werden kann.

Der Probenbehälter weist einen oberen Bund auf, der innerhalb der Probenmulde zugänglich ist, wobei der in die Probenmulde eingesetzte Probenbehälter mit seinem Bund etwa in der Ebene der Fläche an der Oberseite des Schiebers bündig abschließt.

Zugehörig zur Einrichtung ist eine Entnahmevorrichtung mit einer Entnahmezange vorhanden. Die Entnahmevorrichtung ist mit der Entnahmezange, derart in das Gehäuse einsetzbar, dass sich die Entnahmezange in der Ruhestellung des Schiebers axial oberhalb der Probenmulde befindet. Die Entnahmezange kann den oberen Bund des Probenbehälters übergreifen, derart dass der Probenbehälter mit der Entnahmezange in die Probenmulde eingesetzt oder aus dieser entnommen werden kann.

Erfindungsgemäß sind als Entnahmezange an der unteren Stirnseite der Entnahmevorrichtung zwei aufspreizbare Segmente vorgesehen, die zentrisch mindestens einen Ansatz aufweisen. Zugehörig ist in der Entnahmevorrichtung eine Druckstange verschiebbar gelagert, an deren unteren Ende ein Druckkegel vorgesehen ist, der in der Ruhestellung am Ansatz anliegt. Beim Verschieben der Druckstange nach unten wirkt der Druckkegel gegen den Ansatz und die zwei aufspreizbaren Segmente werden zangenartig auseinander gedrückt. In der aufgespreizten Stellung kann die Entnahmezange über den Bund am Probenbehälter greifen.

Die Entnahmevorrichtung kann manuell frei durch einen Entnahmeflansch am Gehäuse einsetzbar ausgebildet sein. In besonderen Fällen kann die Entnahmevorrichtung auch mechanisiert ausgebildet sein. Dazu kann eine mechanische, pneumatisch, hydraulisch oder elektrisch betätigte Einrichtung vorhanden sein, mit der die Entnahmezange an der Entnahmevorrichtung den Probenbehälter greifen und in die bzw. aus der Probenmulde manipulieren kann.

Am Gehäuse ist in vorteilhafter Weise eine untere Öffnung vorgesehen, durch die überschüssige Pulverreste aus dem Raum um den Schieber und der Probenmulde entweichen können. Diese Pulverreste können z.B. in einem angeflanschten Behältnis gesammelt werden.

Insbesondere für den Einsatz der Einrichtung in der pharmazeutischen Industrie werden hohe Ansprüche an die Reinheit und deren Reinigungsmöglichkeit gestellt. Für diesen Zweck ist es vorteilhaft, den Entnahmeflansch mit Leitungen zur Zuführung von flüssigen und/oder gasförmigen Spülmitteln zu verbinden. In entsprechender Weise kann die untere Öffnung am Gehäuse als Spülflansch ausgebildet und mit einer Leitung zur Abführung der über den Entnahmeflansch eingeleiteten flüssigen und/oder gasförmigen Spülmitteln verbunden sein.

Wenn keine Probeentnahme erfolgt, ist es vorteilhaft, am Entnahmeflansch einen Deckel vorzusehen, der das Gehäuse verschließen kann.

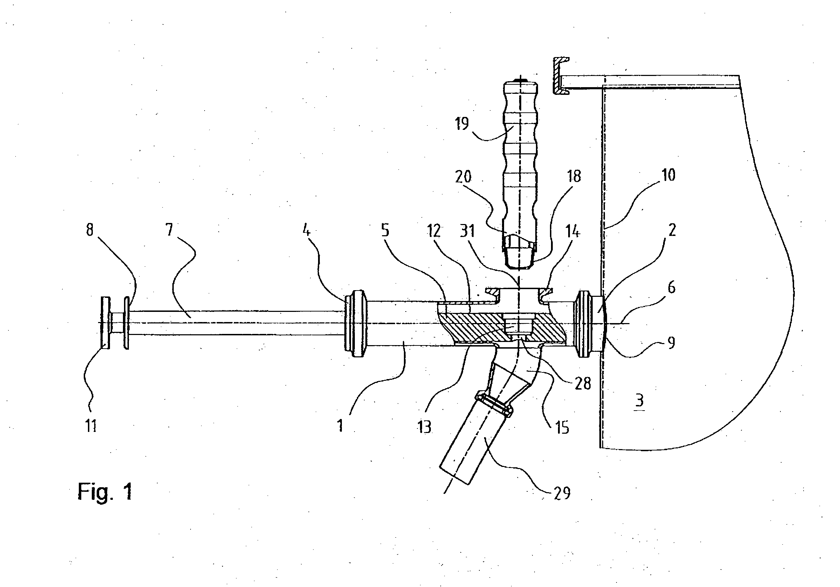

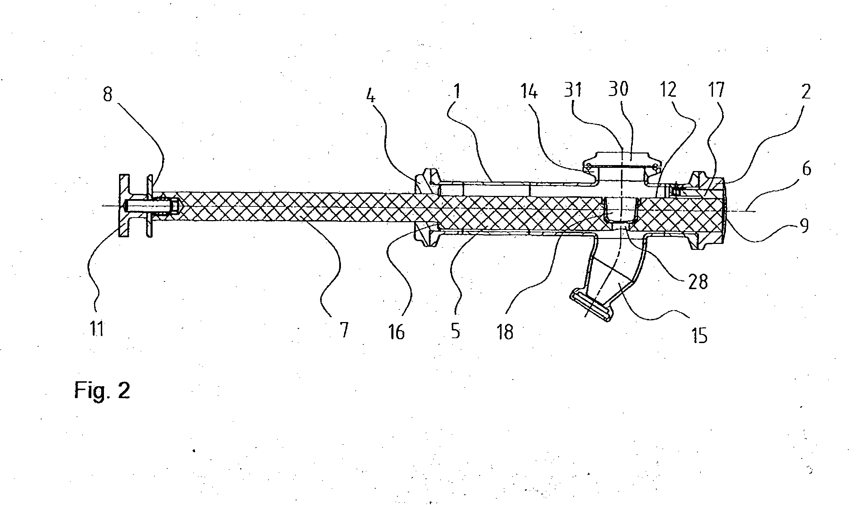

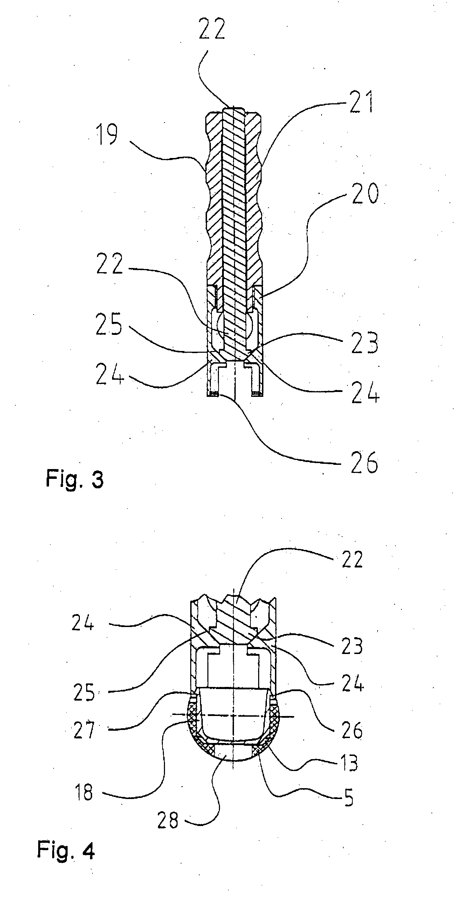

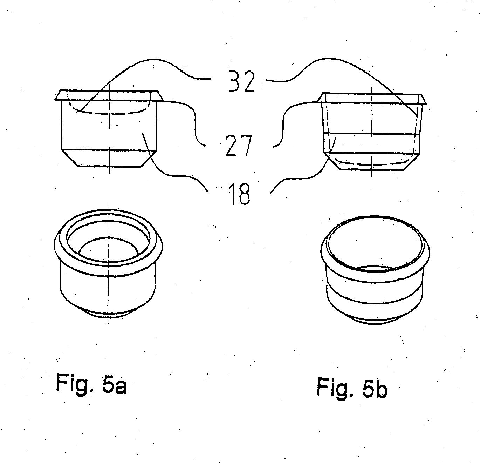

Die Einrichtung wird nachstehend an einem Ausführungsbeispiel näher erläutert. Zugehörig zeigt Figur 1 eine Gesamtübersicht einer erfindungsgemäßen Einrichtung an einer Pulverleitung. Figur 2 zeigt die entsprechende Einrichtung in Alleinstellung und im Schnitt. Figur 3 zeigt eine zugehörige manuell handhabbare Entnahmeeinrichtung. Figur 4 zeigt vergrößert die Entnahmezange am unteren Ende der Entnahmeeinrichtung nach Figur 3 mit einem daran gehalterten Probenbehälter innerhalb der Probenmulde. Figur 5a und 5b zeigen Probenbehälter mit unterschiedlichem Fassungsvermögen.

In Figur 1 als Gesamtübersicht und in Figur 2 als Schnitt ist beispielhaft eine erfindungsgemäße Einrichtung dargestellt. Dabei ist der Grundkörper 1 über einen Flansch 2 an der Wandung einer Pulverleitung 3 angeordnet. Axial gegenüber dem Flansch 2 ist am Grundkörper 1 ein Lagerflansch 4 vorgesehen. Innerhalb des Grundkörpers 1 befindet sich ein Schieber 5, der in der Gehäuseachse 6 verschiebbar ist. Gelagert ist der Schieber 5 im Flansch 2 und im Lagerflansch 4, wobei der Schieber 5 im Bereich des Lagerflansches 4 und außerhalb des Gehäuses 1 als Schubstange 7 ausgebildet ist. Am Übergang vom inneren Abschnitt des Schiebers 5 zur Schubstange 7 ist ein innerer Anschlagbund 16 ausgebildet, der in der Ruhestellung am Lagerflansch 4 anliegt.

Im Ausführungsbeispiel ist an der Schubstange 7 ein Griffelement 11 mit einer äußeren Anschlagsscheibe 8 vorhanden. Bei Bedarf kann statt des Griffelementes 11 auch ein pneumatischer oder elektromotorischer Antrieb vorgesehen sein.

Der mögliche Weg, den die Schubstange 7 innerhalb des Gehäuses 1 verschoben werden kann, wird einerseits durch den inneren Anschlagbund 16 und andererseits durch die äußere Anschlagsscheibe 8 begrenzt, die beidseitig am Lagerflansch 4 anschlagen. Wenn der Schieber 5 mit dem Anschlagbund 16 am Lagerflansch 4 anliegt, befindet sich die Stirnfläche 9 des Schiebers 5 in der Ebene der Innenfläche 10 der Pulverleitung 3. Diese Ebene kann sowohl eine gebogene Rohrfläche als auch eine ebene Fläche einer kastenförmigen Pulverleitung sein.

Der Schieber 5 ist im Flansch 2 über eine Fläche 12 drehfest geführt. Dazu ist in den Flansch 2 eine entsprechende Verdrehsicherung 17 eingebracht. Auf der Oberseite des Schiebers 5, d.h. in der Fläche 12, ist eine Probenmulde 13 eingearbeitet, die zentrisch unten eine Öffnung 28 aufweist.

Am Gehäuse 1 sind oben ein Entnahmeflansch 14 und gegenüberliegend ein Spülflansch 15 vorgesehen. Die Lage der vertikale Achse 31 des Entnahmeflansches 14 entspricht der Lage der zentrischen Achse durch die Probenmulde 13, wenn der Anschlagbund 16 am Lagerflansch 4 anliegt.

Am Spülflansch 15 ist beispielhaft ein Auffangglas 29 vorgesehen, in dem überschüssige Pulverreste aus dem Raum um den Schieber 5 und der Probenmulde 13 gesammelt werden können.

Erfindungsgemäß ist zugehörig zur Einrichtung neben dem Gehäuse 1 mit dem Schieber 5 eine Entnahmevorrichtung 19 mit einer Entnahmezange 20 vorhanden. In Figur 1 ist an der Entnahmezange 20 ein Probenbehälter 18 gehaltert dargestellt. Die Entnahmevorrichtung 19 ist im Ausführungsbeispiel frei handhabbar ausgebildet, d.h. sie wird manuell nur zum Einsetzen und zur Entnahme eines Probenbehälters 18 in die bzw. aus der Probenmulde 13 durch den Entnahmeflansch 14 hindurch geführt. Am Entnahmeflansch 14 kann ein Deckel 30 vorgesehen sein, mit dem das Gehäuse 1 verschlossen werden kann, wenn zeitweise keine Probenentnahme erfolgt.

Die Entnahmevorrichtung 19 ist in Figur 3 und ausschnittsweise in Figur 4 vergrößert dargestellt. Die Entnahmevorrichtung 19 besteht aus einem Griff 21 mit einer unten angeordneten Entnahmezange 20. Durch den oberen Abschluss des Griffes 21 hindurch ist eine Druckstange 22 mit einem unteren Druckkegel 23 geführt und gehaltert. Als Entnahmezange 20 sind zwei elastische Abschnitte 24 vorgesehen. Innen im Bereich der beiden elastischen Abschnitte 24 befindet sich ein bundartiger Ansatz 25, an dem im Ruhezustand der Druckkegel 23 anliegt. An den stirnseitigen Enden der elastischen Abschnitte 24 befinden sich Einkerbungen 26.

Figur 4 zeigt die Entnahmezange 20 in der Position, bei der sie mit den Einkerbungen 26 innerhalb der Probenmulde 13 einen oberen Bund 27 des Probenbehälters 18 übergreift.

Die Figuren 5a und 5b zeigen je einen Probenbehälter 18, wobei die Figur 5a beispielhaft einen Probenbehälter 18 mit einem Fassungsvermögen von 1cm³ und Figur 5b einen Probenbehälter 18 mit einem Fassungsvermögen von 4cm³ zeigen. Der Innenraum ist jeweils mit gestrichelter Linie 32 markiert.

Die an einer Einrichtung eingesetzten Probenbehälter 18 haben dabei alle die gleichen äußeren Abmessungen, jedoch kann der innere Raum, d.h. das Fassungsvermögen des Probenbehälters 18 in seiner Größe frei an die unterschiedlichen technologischen Anforderungen angepasst sein.

Die Stirnseite eines in die Probenmulde 13 eingesetzten Probenbehälters 18, liegt etwa in der Ebene der Fläche 12 innerhalb des Schiebers 5, so dass der Schieber 5 mit dem Probenbehälter 18 gut durch den Flansch 2 geschoben werden kann.

Im Ausführüngsbeispiel besteht der Probenbehälter 18 aus Edelstahl und die Entnahmezange 20 mit den beiden elastische Abschnitten 24 aus Polytetrafluorethylen (PTFE). Damit sind die beiden elastischen Abschnitte 24 auch materialspezifisch elastisch und können den Bund 27 gut übergreifen.

Bei Bedarf kann an der Schubstange 7 auch eine Rasterung oder Markierung vorgesehen sein, mit der bei der Probenentnahme eine spezifische Position des Probenbehälters 18 innerhalb der Pulverleitung 3 eingestellt werden kann. D.h. entsprechend einer technologischen Vorgabe kann Pulver z.B. vom Randbereich oder der Mitte der Pulverleitung 3 entnommen werden.

Nachfolgend wird die Einrichtung zur Entnahme von Proben in der Anwendung näher beschrieben. Innerhalb der Pulverleitung 3 wird ein Pulver oder Granulat einer sich leicht entmischenden Produktmischung im freien Fall befördert. Entsprechend spezifischer technologischer Vorgaben sollen periodisch Proben entnommen werden und das Mischungsverhältnis sowie die Homogenität der Produktmischung überprüft werden.

Zur Entnahme der Probe wird ein Probenbehälter 18 mit einem technologisch vorgesehenen Fassungsvermögen ausgewählt und mit der Entnahmezange 20 an der Entnahmevorrichtung 19 erfasst. Beim Einsetzen eines Probenbehälters 18 in die Probenmulde 13 wird die Entnahmevorrichtung 19 außerhalb der Einrichtung auf den Probenbehälter 18 aufgesetzt und derart axial aufgedrückt, dass die Druckstange 22 mit seinem unteren Druckkegel 23 gegen den bundartigen Ansatz 25 an den elastischen Abschnitten 24 wirkt und die elastischen Abschnitte 24 auseinander gedrückt werden, bis der Bund 27 des Probenbehälters 18 in die Einkerbung 26 einrastet. Der praktisch erforderliche Hub der Druckstange 22 beträgt etwa 2 mm.

Danach wird die Entnahmevorrichtung 19 mit dem Probenbehälter 18 durch den Entnahmeflansch 14 in das Gehäuse 1 eingeführt und der Probebehälter 18 in die Probenmulde 13 eingesetzt. Danach werden durch Drücken der Druckstange 22 die elastische Abschnitte 24 wieder gespreizt und die Entnahmevorrichtung 19 wird vom Probenbehälter 18 gelöst.

Zur Entnahme der Probe wird der Schieber 5 über das Griffelement 11 an der Schubstange 7 in der Gehäuseachse 6 verschoben, bis der Anschlagsscheibe 8 am Lagerflansch 4 anschlägt. In dieser Position befindet sich der Probenbehälter 18 in der Probenmulde 13 zentrisch innerhalb des frei fallenden Pulverstromes in der Pulverleitung 3.

Bei Bedarf kann die Schubstange 5 auch nur bis eine bestimmte Markierung geschoben werden, die der Lage des Probenbehälter 18 in einem radialen Abstand von der Mitte der Pulverleitung 3 entspricht.

In kurzer Zeit ist der Probenbehälter 18 gefüllt und der Schieber 5 kann zurück gezogen werden. Dabei wird Pulver, welches sich oberhalb des Probenbehälters 18 befindet am Flansch 2 abgestriffen, so dass der Probenbehälter 18 entsprechend seines Volumens genau bis zum Rand gefüllt ist.

Der Schieber 5 wird wieder zurück gezogen, bis der Anschlagbund 16 am Lagerflansch 4 anliegt. Die Entnahmevorrichtung 19 wird auf den Probenbehälter 18 aufgesetzt und die Entnahmezange 20 mit der Druckstange 22 gespreizt, bis der Bund 27 des Probenbehälters 18 in die Einkerbung 26 einrastet. Danach wird die Entnahmevorrichtung 19 mit dem gefüllten Probenbehälter 18 durch den Entnahmeflansch 14 entfernt und zur Analysestation gebracht werden. Gegebenenfalls können auch mehrere Proben nacheinander entnommen und gesammelt zur Analysestation gebracht werden.

Unabhängig vom Prozess der Probenentnahme ist es in der Praxis erforderlich, den Innenraum des Gehäuses 1 mit dem Schieber 5 periodisch zu reinigen. Dazu kann ein flüssiges und/oder gasförmiges Reinigungsmittel über den Entnahmeflansch 14 und durch das Gehäuse 1 hindurch zum Spülflansch 15 geleitet werden.

Insbesondere bei der Anwendung der Einrichtung in der pharmazeutischen Industrie muss diese hohen Standards zur Reinheit entsprechen. Für derartige Anwendungen kann die Einrichtung in geeigneter Weise mit Leitungen zur Zu- und Abführung der Reinigungsmittel verbunden werden. Beispielsweise kann der Innenraum des Gehäuses 1 vom Entnahmeflansch 14 zum Spülflansch 15 erst mit einem flüssigen Reinigungsmittel und danach mit einem Trocknungsgas gespült werden. Danach steht die Einrichtung mit hoher Reinheit für die nächste Probenahme einer gleichartigen oder einer anderen Produktprobe zur Verfügung.

### Liste der verwendeten Bezugszeichen

- 1: Grundkörper
- 2: Flansch
- 3: Pulverleitung
- 4: Lagerflansch
- 5: Schieber
- 6: Gehäuseachse
- 7: Schubstange
- 8: Anschlagsscheibe
- 9: Stirnfläche
- 10: Innenfläche
- 11: Griffelement
- 12: Fläche
- 13: Probenmulde
- 14: Entnahmeflansch
- 15: Spülflansch
- 16: Anschlagbund
- 17: Verdrehsicherung
- 18: Probenbehälter
- 19: Entnahmevorrichtung
- 20: Entnahmezange
- 21: Griff
- 22: Druckstange
- 23: Druckkegel
- 24: elastische Abschnitte
- 25: bundartiger Ansatz
- 26: Einkerbung
- 27: Bund
- 28: Öffnung
- 29: Auffangglas
- 30: Deckel
- 31: vertikale Achse
- 32: Linie

## Patentansprüche

1. Einrichtung zur Entnahme von Proben aus einem Pulverstrom, der in einer Pulverleitung (3) geführt wird, bestehend aus einem außen an der Pulverleitung (3) angeordneten Gehäuse (1) mit einer zur Längsachse der Pulverleitung (3) radialen Gehäuseachse (6), einem Schieber (5), der in der Gehäuseachse (6) verschiebbar gelagert ist, dessen Stirnfläche (9) in der Ruhestellung bündig zur Innenwandung der Pulverleitung (3) liegt und der an seinem Umfang, zum Pulverstrom oben, eine Probenmulde (13) aufweist, einer Schubeinrichtung, die geeignet ist, den Schieber (5) in der Gehäuseachse (6) zu verschieben, derart dass die Probenmulde (13) in den Pulverstrom bewegt werden kann, wobei der Schieber (5) mindestens im Bereich von der Probenmulde (13) bis zur Stirnfläche (9) passend zu einer entsprechenden Öffnung in der Pulverleitung (3) eine Fläche (12) aufweist, **dadurch gekennzeichnet, dass** ein Probenbehälter (18) vorhanden ist, der in der Probenmulde (13) lagerbar ist, und eine Entnahmevorrichtung (19) mit einer Entnahmezange (20) vorhanden ist, die in das Gehäuse (1) derart einführbar ist, dass sich die Entnahmezange (20) in der Ruhestellung des Schiebers (5) axial oberhalb der Probenmulde (13) befindet und auf den Probenbehälter (18) aufsetzbar und mit diesem verbindbar ist.

2. Einrichtung nach Anspruch 1, **dadurch gekennzeichnet, dass** an der unteren Stirnseite der Entnahmevorrichtung (19) als Entnahmezange (20) zwei aufspreizbare elastische Abschnitte (24) vorhanden sind, die zentrisch einen Ansatz (25) aufweisen, und dass in der Entnahmevorrichtung (19) eine Druckstange (22) verschiebbar gelagert ist, an deren unteren Ende ein Druckkegel (23) angeordnet ist, der in der Ruhestellung am Ansatz (25) anliegt.

3. Einrichtung nach Anspruch 1 oder 2, **dadurch gekennzeichnet, dass** am Gehäuse (1) ein Entnahmeflansch (14) vorhanden ist, durch den die Entnahmezange (20) bis zur Probenmulde (13) einführbar ist.

4. Einrichtung nach Anspruch 1 oder 2, **dadurch gekennzeichnet, dass** am Probenbehälter (18) ein Bund (27) und passend dazu an den elastischen Abschnitten (24) Einkerbungen (26) vorhanden sind, derart dass ein Probenbehälter (18) an der Entnahmezange (20) haltbar ist.

5. Einrichtung nach Anspruch 3, **dadurch gekennzeichnet, dass** am Entnahmeflansch (14) ein Deckel (30) vorhanden ist.

6. Einrichtung nach Anspruch 3, **dadurch gekennzeichnet, dass** am Gehäuse (1) unten ein Spülflansch (15) vorhanden ist.

7. Einrichtung nach Anspruch 6, **dadurch gekennzeichnet, dass** der Entnahmeflansch (14) mit Leitungen zur Zuführung und der Spülflansch (15) mit einer Leitung zur Abführung von flüssigen und/oder gasförmigen Spülmitteln verbunden sind.

8. Einrichtung nach Anspruch 1, **dadurch gekennzeichnet, dass** eine mechanische Einrichtung vorhanden ist, mit der die Entnahmezange (20) an der Entnahmevorrichtung (19) den Probenbehälter (18) greifen und in die bzw. aus der Probenmulde (13) manipulieren kann.

## Claims

1. Device for taking samples from a powder stream which is guided within a powder line (3), consisting of a housing (1) arranged externally on the powder line (3), with a housing axis (6) which is radial with respect to the longitudinal axis of the powder line (3); a slide (5) which is arranged slidably in the housing axis (6) and whose end face (9), in the rest position, lies flush with the internal wall of the powder line (3) and which has on its circumference, upwards towards the powder stream, a sample hollow (13); a sliding device which is designed to slide the slide (5) in line with the housing axis (6) such that the sample hollow (13) can be moved into the powder stream, wherein the slide (5) has, at least in the region from the sample hollow (13) to the end face (9) matching a corresponding opening in the powder line (3), a surface (12), **characterized in that** a sample container (18) is present which can be mounted in the sample hollow (13), and a removal device (19) with a removal tongs (20) is present which can be introduced into the housing (1) in such a manner that the removal tongs (20), in the rest position of the slide (5), is located axially above the sample hollow (13) and can be placed onto the sample container (18) and can be connected to the latter.

2. Device according to Claim 1, **characterized in that**, on the lower end face of the removal device (19), as removal tongs (20), there are two spreadable elastic sections (24) which have, centrally, a base (25), and **in that** a pressure rod (22) is mounted slidably in the removal device (19), at the lower end of which rod is arranged a pressure cone (23) which, in the rest position, rests against the base (25).

3. Device according to Claim 1 or 2, **characterized in that** a removal flange (14), through which the removal tongs (20) can be inserted as far as the sample hollow (13), is present on the housing (1).

4. Device according to Claim 1 or 2, **characterized in that** a collar (27) is present on the sample container (18) and matching notches (26) are present on the elastic sections (24), such that a sample container (18) can be held by the removal tongs (20).

5. Device according to Claim 3, **characterized in that** a cover (30) is present on the removal flange (14).

6. Device according to Claim 3, **characterized in that** a flushing flange (15) is present on the lower part of the housing (1).

7. Device according to Claim 6, **characterized in that** the removal flange (14) is connected to lines for supplying liquid and/or gaseous flushing media, and the flushing flange (15) is connected to a line for discharging these media.

8. Device according to Claim 1, **characterized in that** there is a mechanical device by means of which the removal tongs (20) on the removal device (19) grip the sample container (18) and can move the latter into or out from the sample hollow (13).

## Revendications

1. Dispositif pour prélever des échantillons dans un flux de poudre qui est guidé dans une conduite de poudre (3), constitué d'un boîtier (1) disposé à l'extérieur sur la conduite de poudre (3) avec un axe de boîtier (6) radial par rapport à l'axe longitudinal de la conduite de poudre (3), d'un coulisseau (5) qui est supporté de manière déplaçable dans l'axe de boîtier (6), dont la surface frontale (9), dans la position de repos, se situe en affleurement par rapport à la paroi interne de la conduite de poudre (3) et qui présente, à sa périphérie, en haut par rapport au flux de poudre, un creux d'échantillon (13), d'un dispositif de poussée qui est approprié pour déplacer le coulisseau (5) dans l'axe de boîtier (6) de telle sorte que le creux d'échantillon (13) puisse être déplacé dans le flux de poudre, le coulisseau (5), au moins dans la région allant du creux d'échantillon (13) jusqu'à la surface frontale (9), présentant une surface (12) en ajustement avec une ouverture correspondante dans la conduite de poudre (3), **caractérisé en ce qu'**il est prévu un récipient d'échantillon (18) qui peut être supporté dans le creux d'échantillon (13), et un dispositif de prélèvement (19) avec une pince de prélèvement (20), qui peut être introduite dans le boîtier (1) de telle sorte que la pince de prélèvement (20), dans la position de repos du coulisseau (5), se trouve axialement au-dessus du creux d'échantillon (13) et puisse être posée sur le récipient d'échantillon (18) et être connectée à celui-ci.

2. Dispositif selon la revendication 1, **caractérisé en ce qu'**au niveau du côté frontal inférieur du dispositif de prélèvement (19) sont prévues, en tant que pince de prélèvement (20), deux portions élastiques écartables (24) qui présentent centralement un saillie (25) et **en ce que** dans le dispositif de prélèvement (19) est supportée de manière déplaçable une tige de pression (22) au niveau de l'extrémité inférieure de laquelle est disposé un cône de pression (23) qui s'applique dans la position de repos contre la saillie (25).

3. Dispositif selon la revendication 1 ou 2, **caractérisé en ce qu'**une bride de prélèvement (14) est prévue au niveau du boîtier (1), par le biais de laquelle la pince de prélèvement (20) peut être introduite jusqu'au creux d'échantillon (13).

4. Dispositif selon la revendication 1 ou 2, **caractérisé en ce qu'**un épaulement (27) et des encoches (26) s'adaptant à celui-ci au niveau des portions élastiques (24) sont prévus sur le récipient d'échantillon (18) de telle sorte qu'un récipient d'échantillon (18) puisse être retenu sur la pince de prélèvement (20).

5. Dispositif selon la revendication 3, **caractérisé en ce qu'**un couvercle (30) est prévu au niveau de la bride de prélèvement (14).

6. Dispositif selon la revendication 3, **caractérisé en ce qu'**une bride de rinçage (15) est prévue en dessous au niveau du boîtier (1).

7. Dispositif selon la revendication 6, **caractérisé en ce que** la bride de prélèvement (14) est connectée à des conduites pour l'alimentation et la bride de rinçage (15) est connectée à une conduite pour l'évacuation d'agents de rinçage liquides et/ou gazeux.

8. Dispositif selon la revendication 1, **caractérisé en ce qu'**un dispositif mécanique est prévu, avec lequel la pince de prélèvement (20) peut saisir le récipient d'échantillon (18) au niveau du dispositif de prélèvement (19) et le manipuler dans le creux d'échantillon (13) ou hors de celui-ci.
